(19) Europäisches Patentamt
European Patent Office
Office européen des brevets

(11) **EP 2 105 086 B1**

(12) **EUROPEAN PATENT SPECIFICATION**

(45) Date of publication and mention
of the grant of the patent:
**20.07.2011 Bulletin 2011/29**

(51) Int Cl.:
*A61B 1/05* (2006.01)    *A61B 5/00* (2006.01)

(21) Application number: **09004499.1**

(22) Date of filing: **27.03.2009**

(54) **Electronic endoscope apparatus**

**Elektronisches Endoskopgerät**

**Appareil d'endoscope électronique**

(84) Designated Contracting States:
**DE FR**

(30) Priority: **28.03.2008 JP 2008087329**

(43) Date of publication of application:
**30.09.2009 Bulletin 2009/40**

(73) Proprietor: **FUJIFILM Corporation**
**Tokyo 106-0031 (JP)**

(72) Inventor: **FUJITA, Hiroshi**
**Saitama-shi**
**Saitama-ken (JP)**

(74) Representative: **Klunker . Schmitt-Nilson . Hirsch**
**Patentanwälte**
**Destouchesstrasse 68**
**80796 München (DE)**

(56) References cited:
**EP-A- 1 813 185    EP-A- 1 880 661**
**EP-A- 1 994 875**

**Description**

BACKGROUND OF THE INVENTION

Field of the Invention

The present invention relates generally to an electronic endoscope apparatus, and more particularly to an electronic endoscope apparatus capable of displaying a spectrum of a specified region of interest by arithmetically processing an image signal representing a color image of an observation object.

Description of the Related Art

Recently, in the field of electronic endoscope systems with a solid state imaging device, those that performs spectroscopic imaging using combined narrow bandpass filters based on the spectral reflection factors at digestive organs, such as gastric mucosa and the like, i.e., electronic endoscope systems having therein narrow-band filters (Narrow Band Imaging, NBI) have been drawing attention. The system includes three narrow (wavelength) bandpass filters, instead of frame sequential type R (red), G (green), and B (blue) rotation filters. In the system, illumination light is sequentially outputted through the narrow bandpass filters, and three signals obtained by the illumination light are processed by changing the weighting in the similar manner as in the R, G, B (RGB) signals, thereby generating a spectroscopic image. According to such spectroscopic images, microscopic structures and the like, not obtainable in the past, may be detected in digestive organs, such as stomach and large intestine.

In a so-called simultaneous type endoscope system that uses microscopic mosaic color filters disposed on the solid state imaging device, unlike the frame sequential type endoscope system that uses narrow bandpass filters, a method for generating a spectroscopic image based on image signals obtained by imaging an observation object while emitting white light on the object is proposed as described, for example, in Japanese Unexamined Patent Publication Nos. 7(1995)-096005 and 2003-093336, and a book "Introduction to Multi-spectral Imaging" by Yoichi MIYAKE, University of Tokyo Press, 2006, pp. 21-35, and pp. 171-173 (ISBN4-13-062141-6). The method obtains the relationship between color sensitivity characteristic of each of RGB converted to numeric data and spectroscopic characteristic of a specific narrow bandpass converted to numeral data as matrix data (a set of coefficients), and spectroscopic image signals are obtained by estimating a spectroscopic image which would be obtained through narrow bandpass filters by performing arithmetic operations between the matrix data and the RGB signals. Generation of a spectroscopic image by such arithmetic operations does not require a plurality of different filters corresponding to intended wavelength ranges and replacement thereof, so that the system may be kept small with reduced cost.

In the mean time, a method that applies scattering imaging, a technology for imaging scattering features of a living tissue, to determine whether or not a lesion is found in the living tissue from the image is also proposed as described, for example, in Japanese Unexamined Patent Publication No. 2004-202217. In the scattering imaging described in Japanese Unexamined Patent Publication No. 2004-202217, it is also described that a reflection spectrum is estimated from multi-band image data, such as R, G, and B data, in the course of processing, but the display of the estimated reflection spectrum is not described at all.

In actual clinical field, it is also proposed to compare a reflection spectrum of a region of interest to a reflection spectrum of a corresponding region known to be normal, thereby determining whether the region of interest is normal or not, in addition to referring to a spectroscopic image obtained by the arithmetic operation described above or the like. Heretofore, however, it has taken a lot of time and effort to measure and display such reflection spectrum, and any of the conventional technologies described above is not able to display such reflection spectrum.

The present invention has been developed in view of the circumstances described above, and it is an object of the present invention to provide an electronic endoscope apparatus capable of displaying a reflection spectrum of a desired region of interest of an observation object easily.

EP 1 994 875 A1 discloses an endoscope according to the preamble of claim 1.

SUMMARY OF THE INVENTION

An electronic endoscope apparatus of the present invention is an apparatus which includes a light source for emitting white light on an observation object, a color imaging device for imaging the observation object illuminated by the white light, and an image display means for displaying a color image of the observation object from RGB three color image signals based on output of the color imaging device, in which the apparatus further includes:

a position specifying means for specifying a desired region of interest in the color image displayed on the image display means;

a calculation means for estimating a reflection spectrum of the observation object with respect to the region of interest specified by the position specifying means by performing arithmetic operations between RGB three color image signals of the region of interest and predetermined matrix data based on foresight information; and

a spectrum display means for displaying the reflection spectrum by receiving output of the calculation means.

Preferably, the spectrum display means doubles as an image display means for displaying a color image of an observation object.

Further, in the electronic endoscope apparatus of the present invention, the position specifying means is configured so as to be capable of specifying a plurality of points in a region of interest, and the calculation means is configured to perform a principal component analysis on a plurality of estimated reflection spectra with respect to each of the plurality of points and to output information representing a reflection spectrum of the first principal component obtained by the analysis to the spectrum display means.

Further, in the electronic endoscope apparatus of the present invention, it is preferable that a storage means is further provided for storing information representing the reflection spectrum of the observation object with respect to the region of interest obtained by the calculation means, and the spectrum display means is configured so as to be capable of displaying the reflection spectrum represented by the information sent from the storage means together with another reflection spectrum newly estimated by the calculation means.

Still further, in the electronic endoscope apparatus of the present invention, it is preferable that a spectroscopic image generation circuit is further provided for generating spectroscopic image signals representing a spectroscopic image of a specified wavelength by performing arithmetic operations between the RGB three color image signals based on output of the color imaging device and predetermined matrix data.

The estimation of reflection spectra itself is described, for example, in a literature "Estimation of Spectral Reflectances from Multi-Band Images by Multiple Regression Analysis" by N. TSUMURA et al., Optics 1998, 27 (7), pp. 384-391, but heretofore visualizing and displaying such estimated reflection spectra as images in electronic endoscope apparatuses have not been performed at all.

The electronic endoscope apparatus of the present invention is configured such that a reflection spectrum of an observation object with respect to a region of interest specified by the position specifying means is estimated by performing arithmetic operations between RGB three color image signals of the region of interest and predetermined matrix data based on foresight information, and the estimation result is displayed on the spectrum display means, so that a reflection spectrum of an observation object may be displayed easily.

In particular, where the image display means doubles as the spectrum display means, the cost of the electronic endoscope apparatus may be kept low.

In the electronic endoscope apparatus of the present invention, where the position specifying means is configured so as to be capable of specifying a plurality of points in a region of interest and the calculation means is configured to perform a principal component analysis on a plurality of estimated reflection spectra with respect to each of the plurality of points and to output information representing a reflection spectrum of the first principal component obtained by the analysis to the spectrum display means, a highly reliable reflection spectrum may be displayed.

Further, in the electronic endoscope apparatus of the present invention, where a storage means is further provided for storing information representing the reflection spectrum of the observation object with respect to the region of interest obtained by the calculation means, and the spectrum display means is configured so as to be capable of displaying the reflection spectrum represented by the information sent from the storage means together with another reflection spectrum newly estimated by the calculation means, reflection spectra of a plurality of corresponding regions of interest may be displayed for comparison. In this case, for example, when a reflection spectrum which is distinctly different from a reflection spectrum known to be normal is displayed, the region of interest from which the latter reflection spectrum is obtained may be determined to be a lesion, so that advantageous effects in assisting in medical diagnosis are significant.

Still further, in the electronic endoscope apparatus of the present invention, where a spectroscopic image generation circuit is further provided for generating spectroscopic image signals representing a spectroscopic image of a specified wavelength by performing arithmetic operations between the RGB three color image signals based on output of the color imaging device and predetermined matrix data, it is possible to find out a seemingly abnormal region of interest from a displayed reflection spectrum, and then to generate and display a detailed spectroscopic image of the region of interest. This may avoid the generation of many useless spectroscopic images and the efficiency of medical diagnosis may be improved.

BRIEF DESCRIPTION OF THE DRAWINGS

3

Figure 1 is a block diagram of an electronic endoscope apparatus according to an embodiment of the present invention, illustrating the configuration thereof.

Figure 2 illustrates a layout of the operation panel of the processor unit included in the electronic endoscope apparatus shown in Figure 1 and example wavelength sets.

Figures 3A to 3D illustrate the wavelength information display area of a monitor of the electronic endoscope apparatus shown in Figure 1 and example displays.

Figure 4 is a graph illustrating an example of wavelength ranges of a spectroscopic image with spectroscopic sensitivity characteristics of a primary color CCD.

Figure 5 is a graph illustrating an example of wavelength ranges of a spectroscopic image with a reflection spectrum of a biological object.

Figure 6 illustrates wavelength switching performed by a wavelength change switch of the electronic endoscope apparatus shown in Figure 1.

Figure 7 illustrates wavelength sets to be selected in monochrome mode in the electronic endoscope shown in Figure 1.

DESCRIPTION OF THE PREFERRED EMBODIMENTS

Hereinafter, an embodiment of the present invention will be described in detail with reference to the accompanying drawings.

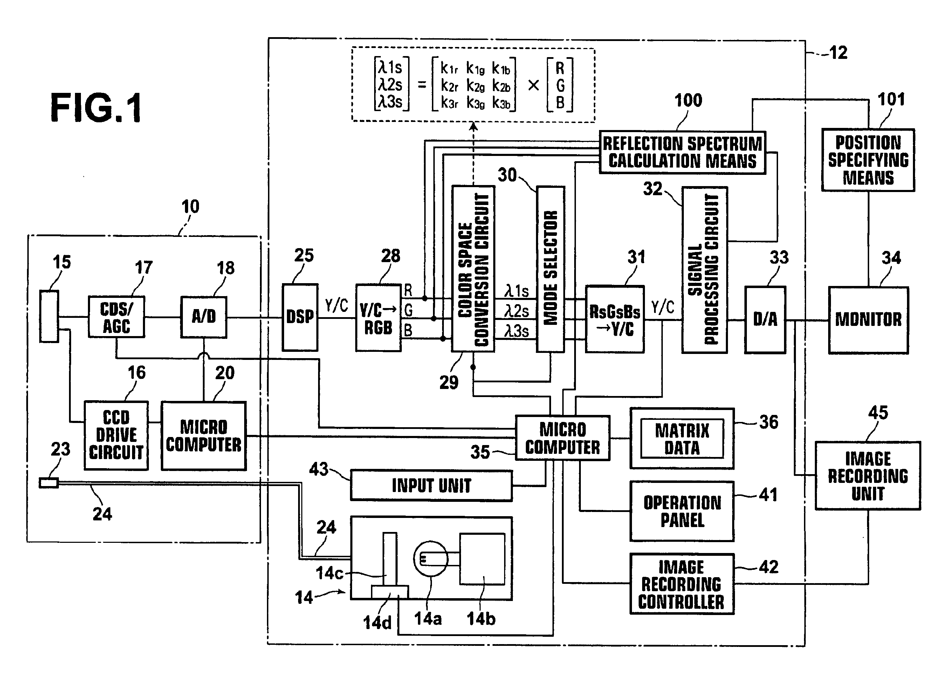

Figure 1 illustrates a basic configuration of an electronic endoscope apparatus according to an embodiment of the present invention. The electronic endoscope apparatus according to the present embodiment is set to either one of two modes, on of which is a mode in which a spectroscopic image of an observation object formed in a manner to be described later is displayed and the other of which is a mode in which an ordinary image of the observation object is displayed. In addition, the apparatus is capable of estimating a reflection spectrum of an intended region of interest of the observation object and displaying the estimated reflection spectrum in an area of a display means in either of the modes.

First, a spectroscopic image display mode and a configuration therefor will be described. As shown in Figure 1, the electronic endoscope apparatus of the present embodiment includes scope 10, a main body of endoscope, and processor unit 12 to which scope 10 is removably connected. Processor unit 12 includes a light source unit 14 that emits white light. Scope 10 has illumination window 23 at the distal end thereof, and a second end of light guide 24 whose first end is connected to light source unit 14 faces illumination window 23. Note that light source unit 14 maybe provided separately from processor unit 12.

Scope 10 further includes CCD 15, a solid-state imaging device, at the distal end thereof. As for CCD 15, for example, a primary color CCD having RBG primary color filters on the imaging plane is used.

Connected to CCD 15 are CCD drive circuit 16 that generates a drive pulse based on a synchronization signal and CDS/AGC (correlated double sampling/automatic gain control) circuit 17 that samples and amplifies image (picture) signals outputted from CCD 15. Connected to CDS/AGC circuit 17 is A/D converter 18 that digitizes analog output of the CDS/AGC circuit 17. Scope 10 further includes microcomputer 20 that controls various circuits in the scope and communication with processor unit 12.

Processor unit 12 includes DSP (digital signal processor) 25 that performs various types of image processing on an image signal digitized by A/D converter 18. DSP 25 generates and outputs Y/C signals that include a luminance (Y) signal and a color difference [C(R-Y, B-Y)] signal from the image signal and first color conversion circuit 28 is connected thereto. First color conversion circuit 28 converts the Y/C signals outputted from DSP 25 to RGB three color image signals. Note that DSP 25 may be provided in scope 10.

Serially connected on the latter stage of first color conversion circuit 28 are: color space conversion circuit 29 that performs a matrix operation for generating a spectroscopic image and outputs an image signal representing a spectroscopic image of selected wavelength ranges $\lambda1$, $\lambda2$, and $\lambda3$; mode selector 30 that selects either one of monochrome mode in which a spectroscopic image of one narrow wavelength range is generated and three-color mode in which a spectroscopic image of three wavelength ranges is generated; second color conversion circuit 31 that receives image signals of three (or one) wavelength ranges $\lambda1s$, $\lambda2s$, and $\lambda3s$ as Rs, Gs, and Bs signals in order to perform RGB signal-comparable processing and converts the Rs, Gs, and Bs signals to Y/C signals; and D/A converter 33. D/A converter 33 at the final stage is connected to monitor 34, for example, a liquid crystal display or a CRT display, and image recording unit 35, an optical scan recording unit or the like, disposed outside of the processor unit 12. Note that two color mode in which a spectroscopic image of two wavelength ranges may be set instead of the three-color mode selected by mode selector 30.

Processor unit 12 further includes microcomputer 35 which has functions of communicating with scope 10, controlling various circuits within processor unit 12, inputting matrix (coefficient) data for generating a spectroscopic image to color space conversion circuit 29, and the like. The matrix data for generating a spectroscopic image based on RGB signals are stored in memory 36 in the form of a table. An example of the matrix data stored in memory 36 in the present embodiment is like that shown in Table 1 below.

Table 1

| Parameter | $K_{pr}$ | $K_{pg}$ | $K_{pb}$ |
|---|---|---|---|
| p1 | 0.000083 | -0.00188 | 0.003592 |
| . . . | . . . | . . . | . . . |
| p18 | -0.00115 | 0.000569 | 0.003325 |
| p19 | -0.00118 | 0.001149 | 0.002771 |
| p20 | -0.00118 | 0.001731 | 0.0022 |
| p21 | -0.00119 | 0.002346 | 0.0016 |
| p22 | -0.00119 | 0.00298 | 0.000983 |
| p23 | -0.00119 | 0.003633 | 0.000352 |
| . . . | . . . | . . . | . . . |
| p43 | 0.003236 | 0.001377 | -0.00159 |
| p44 | 0.003656 | 0.000671 | -0.00126 |
| p45 | 0.004022 | 0.000068 | -0.00097 |
| p46 | 0.004342 | -0.00046 | -0.00073 |
| p47 | 0.00459 | -0.00088 | -0.00051 |
| p48 | 0.004779 | -0.00121 | -0.00034 |
| p49 | 0.004922 | -0.00148 | -0.00018 |
| p50 | 0.005048 | -0.00172 | -0.000036 |
| p51 | 0.005152 | -0.00192 | 0.000088 |
| p52 | 0.005215 | -0.00207 | 0.000217 |
| . . . | . . . | . . . | . . . |
| p61 | 0.00548 | -0.00229 | 0.00453 |
| P1 | 1.00000 | 0.00000 | 0.00000 |
| P2 | 0.00000 | 1.00000 | 0.00000 |
| P3 | 0.00000 | 0.00000 | 1.00000 |

The matrix data in Table 1 includes 61 wavelength range parameters (set of coefficients) p1 to p61 obtained by dividing, for example, the wavelength range of 400 to 700nm into segments with a bandwidth of 5nm each, and parameters P1 to P3 for generating an ordinary image. Each of parameters p1 to p61 includes coefficients $k_{pr}$, $k_{pg}$, and $k_{pb}$ (p = 1 to 61) to be used for matrix operation. Parameter P1 includes coefficients (1.00000, 0.00000, and 0.00000), parameter P2 includes coefficients (0.00000, 1.00000, and 0.00000), and parameter P3 includes coefficients (0.00000, 0.00000, and 1.00000).

In the color space conversion circuit 29, the matrix operation of Formula 1 below is performed between the coefficients $k_{pr}$, $k_{pg}$, and $k_{pb}$, and RGB signals outputted from the first color conversion circuit 28, thereby spectroscopic image signals λ1s, λ2s, and λ3s are generated.

【Formula 1】

$$\begin{bmatrix} \lambda 1 \\ \lambda 2 \\ \lambda 3 \end{bmatrix} = \begin{bmatrix} k_{1r} & k_{1g} & k_{1b} \\ k_{2r} & k_{2g} & k_{2b} \\ k_{3r} & k_{3g} & k_{3b} \end{bmatrix} \times \begin{bmatrix} R \\ G \\ B \end{bmatrix}$$

That is, when, for example, 500nm, 620nm, and 650nm are selected respectively as the wavelength ranges $\lambda 1$, $\lambda 2$, and $\lambda 3$ forming a spectroscopic image, the matrix operation is performed using the coefficients (-0.00119, 0.002346, and 0.0016) of parameter p21 corresponding to the center wavelength of 500nm, coefficients (0.004022, 0.000068, and -0.00097) of parameter p45 corresponding to the center wavelength of 620nm, and coefficients (0.005152, -0. 00192, and 0.000088) of parameter p51 corresponding to the center wavelength of 650nm as the coefficients ($k_{pr}$, $k_{pg}$, and $k_{pb}$).

When an instruction to display or record an ordinary image is given in the manner to be described later, the color space conversion circuit 29 performs the matrix operation described above using the coefficients of parameters P1 to P3. Accordingly, in this case, the RGB signals outputted from the first color conversion circuit 28 are outputted as they are from the color space conversion circuit 29.

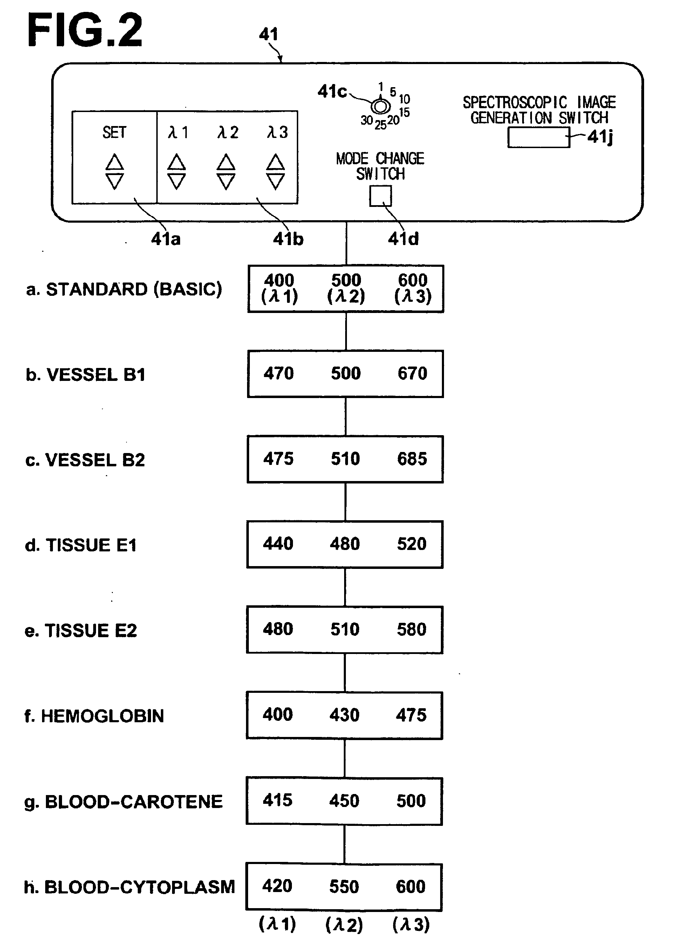

In addition to memory 36, connected to microcomputer 36 are operation panel 41, image recording controller 42, and input unit 43 of a keyboard or the like. Figure 2 illustrates operation panel 41 in detail. The operation panel 41 includes: set selection switch 41a for selecting, for example, one of the wavelength sets of (a) to (h), which are also schematically shown in Figure 2; wavelength selection switches 41b for selecting center wavelengths of wavelength ranges $\lambda 1$, $\lambda 2$, and $\lambda 3$; shift width setting switch 41c for setting a shift width of the wavelength range implemented by wavelength selection switches 41b; monochrome-three color mode change switch 41d for switching between the monochrome mode and three color mode described above; and a spectroscopic image generation switch 41j for instructing generation of a spectroscopic image. Note that the spectroscopic image generation switch 41j may be provided on the side of scope 10.

Processor unit 12 further includes reflection spectrum calculation means 100. Reflection spectrum calculation means 100 receives RGB three color image signals outputted from first color conversion circuit 28. Reflection spectrum calculation means 100 is connected to position specifying means 101 which includes a mouse and control unit, and the like. Position specifying means 101 is connected to monitor 34 described above.

An operation of the electronic endoscope apparatus constructed in the manner as described above will now be described. First, an operation when spectroscopic image generation switch 41j is depressed, i.e., operation for generating a spectroscopic image will be described.

When generating a spectroscopic image, light source unit 14 shown in Figure 1 is activated, and white light emitted therefrom is inputted to light guide 24, which is then outputted from the distal end thereof and emitted on an observation object through illumination window 23. Then, the observation object is imaged by CCD 15 driven by CCD drive circuit 16, and an image signal obtained is outputted therefrom. The image signal is subjected to correlated double sampling and amplification of automatic gain control in the CDS/AGC circuit 17, which is then A/D converted in A/D converter 18 and inputted to DSP 25 of processor unit 12 as a digital signal.

In DSP 25, gamma processing is performed on the signal outputted from scope 10, and color conversion is performed on the signals obtained through the color filters of Mg, Ye, Cy, and G, thereby Y/C signals are generated in the manner as described above. The Y/C signals outputted from the DSP 25 are inputted to the first color conversion circuit 28, where they are converted to RGB signals. The RGB signals are supplied to the color space conversion circuit 29, where a matrix operation is performed between the RGB signals and matrix data to generate a spectroscopic image.

Hereinafter, the matrix operation will be described in detail. When spectroscopic image generation switch 41j on operation panel 41 shown in Figure 2 is depressed, the matrix operation of Formula (1) above is performed to generate a spectroscopic image by the color space conversion circuit 29 using the matrix data stored in the memory 36 and RGB signals of each pixel. That is, in this case, three wavelength ranges $\lambda 1$, $\lambda 2$, and $\lambda 3$ are set through operation panel 41, and the matrix data corresponding to the three wavelength ranges are read out from memory 36 and inputted to color space conversion circuit 29 by microcomputer 35.

If, for example, wavelengths of 500, 620, and 650nm are selected as the three wavelength ranges $\lambda 1$, $\lambda 2$, and $\lambda 3$, the coefficients of parameters p21, p45, and p51 in Table 1, each corresponding to each wavelength, are used, and spectroscopic image signals $\lambda 1s$, $\lambda 2s$, and $\lambda 3s$ are generated from the RGB signals by the matrix operation of Formula 2 below.

【Formula 2】

$$\begin{bmatrix} \lambda 1s \\ \lambda 2s \\ \lambda 3s \end{bmatrix} = \begin{bmatrix} -0.00119 & 0.002346 & 0.0016 \\ 0.004022 & 0.000068 & -0.00097 \\ 0.005152 & -0.00192 & 0.000088 \end{bmatrix} \times \begin{bmatrix} R \\ G \\ B \end{bmatrix}$$

If three color mode is selected by mode selector 30 connected to monochrome-three color mode change switch 41d shown in Figure 2, spectroscopic image signals λ1s, λ2s, and λ3s are inputted to second color conversion circuit 31 as three color image signals Rs, Gs, and Bs respectively. If monochrome mode is selected, any one of spectroscopic image signals λ1s, λ2s, and λ3s is inputted to the second color conversion circuit 31 as Rs, Gs, or Bs signal. Detailed description will be made in the case where the three color mode is selected.

In second color conversion circuit 31, three color image signals Rs, Gs, and Bs are converted to Y/C signals (Y, Rs-Y, Bs-Y), and the Y/C signals are inputted to the monitor 34 and image recording unit 45 through signal processing circuit 32 and D/A converter 33.

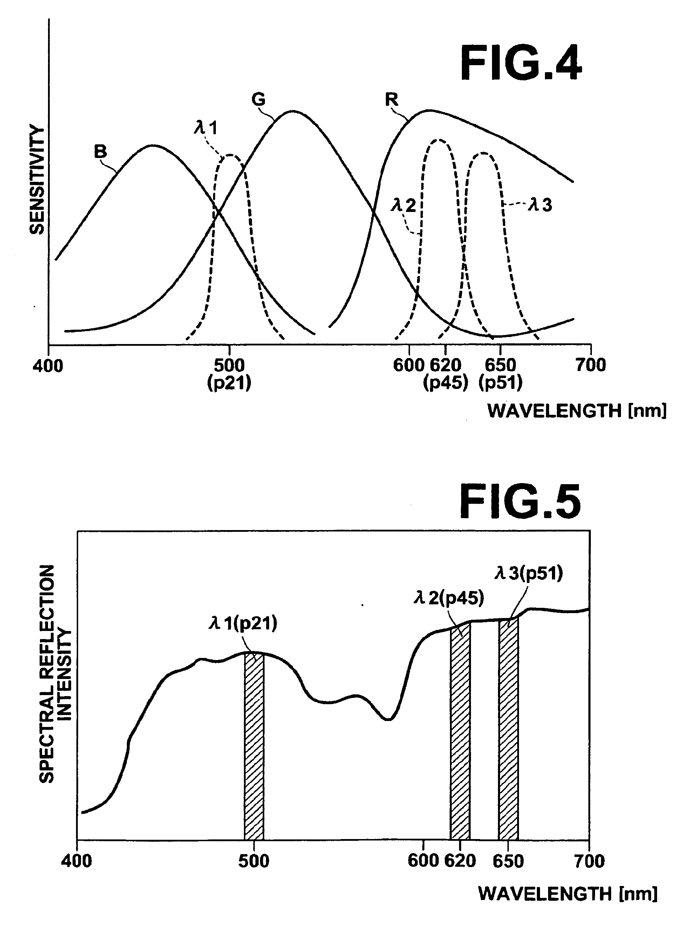

The spectroscopic image displayed on monitor 34 based on the Y/C signals is a color image having color components of wavelength ranges shown in Figures 4 and 5. That is, Figure 4 is a conceptual diagram in which the three wavelength ranges λ1, λ2, and λ3 are superimposed on spectroscopic sensitivity characteristics R, G, and B of the color filters of primary color CCD 15. Figure 5 is a conceptual diagram in which the three wavelength ranges λ1, λ2, and λ3 are superimposed on the reflection spectrum of a biological object. Spectroscopic image signals λ1s, λ2s, and λ3s generated by the example parameters p21, p45, and p51 are color signals within a wavelength range of approximately ± 10nm centered on 500, 620, and 650nm respectively as shown in Figure 5, so that a spectroscopic image (moving picture or still image) formed of color combinations of these three wavelength ranges is displayed or recorded.

Next, selection of wavelength ranges λ1, λ2, and λ3 will be described. In the present embodiment, the following eight wavelength sets are stored in an area of memory 36 shown in Figure 1 as default wavelength sets of λ1, λ2, and λ3 as shown in Figure 2. Namely, a standard (basic) set (a), for example, of 400, 500, and 600 (nm, the same applies hereinafter) ; a vessel B1 set (b) of 470, 500, and 670 for visualizing a vessel; a vessel B2 set (c) of 475, 510, and 685 for also visualizing a vessel; a tissue E1 set (d) of 440, 480, and 520 for visualizing a specific tissue; a tissue E2 set (e) of 480, 510, and 580 for also visualizing a specific tissue; a hemoglobin set (f) of 400, 430, and 475 for visualizing the difference between oxyhemoglobin and deoxyhemoglobin; a blood-carotene set (g) of 415, 450, and 500 for visualizing the difference between blood and carotene; and a blood-cytoplasm set (h) of 420, 550, and 600 for visualizing the difference between blood and cytoplasm.

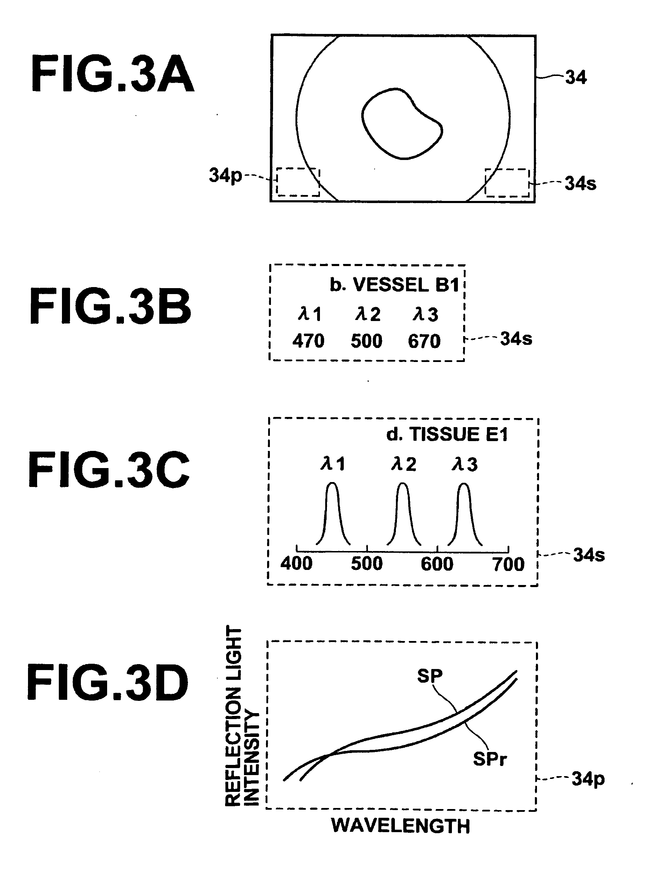

When the electronic endoscope apparatus is initially started up by supplying power after factory shipment, the default wavelength sets described above are selected by the microcomputer 35. Then, when spectroscopic image generation switch 41j on operation panel 41 shown in Figure 2 is depressed, the standard set (a) among the selected wavelength sets is displayed on wavelength information display area 34s of monitor 34 shown in Figure 3A. Here, if the mode change switch 41d is depressed and the three color mode is selected, parameters corresponding to λ1=400, λ2=500, and λ3=600 of the standard set (a) are read out from memory 36, and inputted to the color space conversion circuit 29. The color space conversion circuit 29 performs the matrix operation described above using the parameters inputted in the manner as described above.

Further, the operator of the apparatus, such as a clinician or the like, may freely select other wavelength sets (b) to (h) of the default wavelength sets by operating set selection switch 41a on operation panel 41 shown in Figure 2. Then, the selected wavelength set is displayed on wavelength information display area 34s of monitor 34 shown in Figure 3A by microcomputer 35. At the same time, in this case also, parameters corresponding to λ1, λ2, and λ3 of the selected wavelength set are read out from memory 36 by microcomputer 35 and inputted to color space conversion circuit 29. Color space conversion circuit 29 performs the matrix operation described above using the parameters inputted in the manner as described above.

As shown in Figure 2, set selection switch 41a includes an upward switch having an upward triangular section, and a downward switch having a downward triangular section. Every time the former is depressed, the wavelength sets are sequentially selected in the order of (a), (h), (g) -----, and every time the latter is depressed, the wavelength sets are sequentially selected in the order of (a), (b), (c) -----.

Further, while one of the wavelength set (a) to (h) is selected, the operator may freely change each of the wavelength ranges λ1, λ2, and λ3 by operating corresponding wavelength selection switch 41b. When changing a wavelength range, the wavelength shift width may be changed by shift width setting switch 41c. That is, by rotating the knob of shift width setting switch 41c, a continuous shift or a stepwise shift may be set, like a virtually continuous shift of 1nm width, or a stepwise shift of 5, 10, or 20nm. Note that when the wavelength is changed, for example, by 1nm width, 301 wavelength ranges are set within the range of 400 to 700nm, and matrix data corresponding to the 301 wavelength ranges (p'1 to p'301) are created.

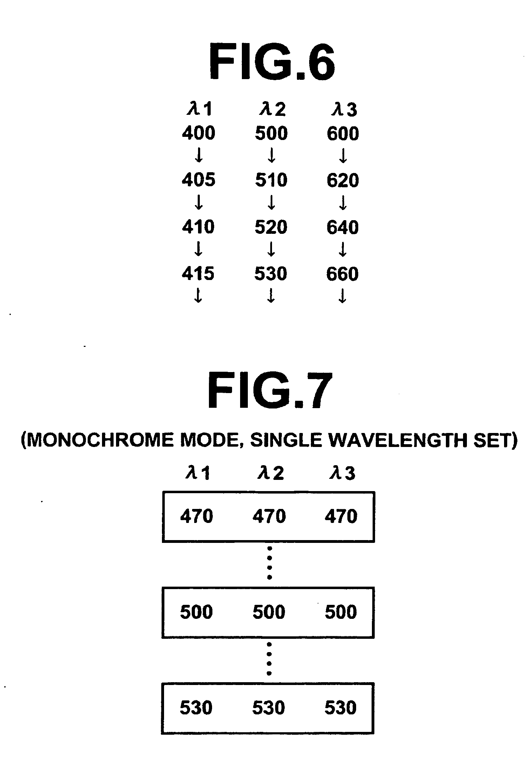

Figure 6 illustrates the selection of the wavelength ranges. When the 5nm width is selected, the wavelength is changed from 400 to 405, and then to 410 as indicated in change of λ1, and if the 10nm width is selected, wavelength is changed from 600 to 620, and then to 640 as indicated in the change of λ3, and these values are displayed on the wavelength information display area 34s of monitor 34.

Figures 3B to 3D illustrate in detail example display statuses of wavelength information display area 34s and reflection spectrum display area 34p, to be described later. In the present embodiment, the wavelength information is displayed on the wavelength information display area 34s provided at the bottom right section or the like of monitor 34, as shown in Figure 3A, through character generation or the like in signal processing circuit 32. That is, values of selected wavelength (nm) are displayed under the characters of λ1, λ2, and λ3 on the wavelength information display area 34s, as shown in Figure 3B. Alternatively, as shown in Figure 3c, the selected wavelength ranges may be visualized by a movable graph with the horizontal axis as the wavelength and the vertical axis as the sensitivity (which corresponds to the graph shown in Figure 4).

The mode change switch 41d shown in Figure 2 is used to perform switching between the monochrome mode and the three color mode. When the apparatus is operated in the three color mode, if the mode change switch 41d is depressed, the apparatus is changed to the monochrome mode, and all wavelength ranges λ1, λ2, and λ3 are set to the same value, like 470, 470, and 470. Then, a common wavelength range is displayed on the monitor 34 as shown in Figure 7. Note that any value may be selected for the common wavelength range by the wavelength selection switches 41b.

Here, in addition to eight default wavelength sets described above, other wavelength sets may be provided and stored in the memory for use, as appropriate, in response to a request from a doctor, the user of the apparatus. Further, some or all of the functions of the switches on the operation panel 41 may be replaced by the key functions of the keyboard.

Next, a mode of displaying an ordinary image of an observation object will be described. While the spectroscopic image is generated and displayed in the manner as described above, if the spectroscopic image generation switch 41j on the operation switch 41 shown in Figure 2 is depressed again, or if the spectroscopic image generation switch 41j is not depressed from the start, the coefficients of parameters P1 to P3 described above are selected as the coefficients of the matrix operation in color space conversion circuit 29, and thereby the RGB signals outputted from first color conversion circuit 28 are outputted as they are from color space conversion circuit 29. Then, the RGB signals are converted to Y/C signals in second color conversion circuit 31, and the Y/C signals are inputted to monitor 34 through signal processing circuit 32 and D/A converter 33. Therefore, an ordinary color image (moving picture or still image) of the observation object is displayed on monitor 34.

In the present embodiment, the output of D/A converter is connected to image recording unit 45 as well as to monitor 34. Thus, if an image recording instruction is given to image recording unit 45 from image recording controller 42, which is controlled by microcomputer 35, a hard copy of an ordinary color image or a spectroscopic image of a scene specified by the image recording instruction is outputted from image recording unit 45.

In conventional endoscope apparatuses, a pigment such as Indigo, Pyoktanin, or the like is dispersed on an observation object, and a tissue colored by the pigment is imaged. If wavelength regions that may visualize a tissue, which may be colored by the dispersal of a pigment, as the wavelength set of λ1, λ2, and λ3, a spectroscopic image which is equivalent to an image obtained by dispersing the pigment may be obtained without requiring the pigment.

Next, a function of estimating and displaying a reflection spectrum of a region of interest will be described. In addition to the matrix data described above, memory 36 has stored therein, as known information, a spectroscopic property matrix of the illumination optical system constituted by light source unit 14, light guide 24 and the like, a spectroscopic property matrix of CCD 15 without the color filters, and a spectroscopic property matrix of the primary color filters of CCD 15 shown below respectively.

【Formula 3】

$$\vec{L} = \begin{pmatrix} L_{\lambda-400} & \cdots & 0 \\ \vdots & \ddots & \vdots \\ 0 & \cdots & L_{\lambda-700} \end{pmatrix}$$

【Formula 4】

$$\vec{O} = \begin{pmatrix} O_{\lambda-400} & \cdots & 0 \\ \vdots & \ddots & \vdots \\ 0 & \cdots & O_{\lambda-700} \end{pmatrix}$$

【Formula 5】

$$\vec{C} = \begin{pmatrix} R_{\lambda-400} & G_{\lambda-400} & B_{\lambda-400} \\ \vdots & \vdots & \vdots \\ R_{\lambda-700} & G_{\lambda-700} & B_{\lambda-700} \end{pmatrix}$$

In the mean time, position specifying means 101 may specify any region of interest on a spectroscopic image or an ordinary image displayed on monitor 34, and information representing the specified region of interest is inputted to reflection spectrum calculation means 100.

Reflection spectrum calculation means 100 obtains reflection spectrum sets of an observation object (test body) represented by Formula 7 below based on the known matrix information shown in Formulae 3 to 5 above and final three color image signal sets with respect to the region of interest outputted from first color conversion circuit 28 shown in Formula 6 below.

【Formula 6】

$$\vec{C_H} = \begin{pmatrix} R_1 & \cdots & R_n \\ G_1 & \cdots & G_n \\ B_1 & \cdots & B_n \end{pmatrix}$$

【Formula 7】

$$\vec{S} = \begin{pmatrix} S_{1\lambda-400} & \cdots & S_{n\lambda-400} \\ \vdots & \ddots & \vdots \\ S_{1\lambda-700} & \cdots & S_{n\lambda-700} \end{pmatrix}$$

Note that "n" in Formulae 6 and 7 above represents the number of points specified in the region of interest, to be described later. That is, the following holds true.

【Formula 8】

$$\vec{C_H} = {}^t(\vec{L} \cdot \vec{O} \cdot \vec{C}) \cdot \vec{S} = {}^t\vec{A} \cdot \vec{S}$$

$$\vec{A} = \vec{L} \cdot \vec{O} \cdot \vec{C}$$

*where,*

$$\vec{A} = \left\{ \begin{array}{ccc} A_{R\lambda=400} & A_{G\lambda=400} & A_{B\lambda=400} \\ \vdots & \vdots & \vdots \\ A_{R\lambda=700} & A_{G\lambda=700} & A_{B\lambda=700} \end{array} \right\}$$

The inverse problem is solved as,

【Formula 9】

$$\vec{S} = (\vec{A} \cdot {}^t\vec{A})^{-1} \cdot \vec{A} \cdot \vec{C_H} = \vec{T} \cdot \vec{C_H}$$

$$\vec{T} = (\vec{A} \cdot {}^t\vec{A})^{-1} \cdot \vec{A}$$

where,

$$\vec{T} = \left\{ \begin{array}{ccc} T_{R\lambda=400} & T_{G\lambda=400} & T_{B\lambda=400} \\ \vdots & \vdots & \vdots \\ T_{R\lambda=700} & T_{G\lambda=700} & T_{B\lambda=700} \end{array} \right\}$$

Therefore, the reflection spectrum sets of the observation object (test body) may be obtained eventually as follows.

【Formula 10】

$$\vec{S} = (\vec{A} \cdot {}^t\vec{A})^{-1} \cdot \vec{A} \cdot \vec{C_H} = \vec{T} \cdot \vec{C_H}$$

In the present embodiment, position specifying means 101 is configured so as to be capable of specifying a plurality of points in a region of interest, and reflection spectrum calculation means 100 is configured to perform a principal component analysis on a plurality of estimated reflection spectra with respect to each of the plurality of points and to input a signal representing a reflection spectrum of the first principal component obtained by the analysis in signal processing circuit 32. Then, based on the signal, the reflection spectrum is displayed in reflection spectrum display area 34p (Figure 3D) of monitor 34.

Hereinafter, the principal component analysis will be described in detail. Here, it is assumed that the number of plural points is represented by "n" as above. The reflection spectra obtained by Formula 10 are assumed to be the following.

【Formula 11】

$$S = \begin{pmatrix} S_{1\lambda-400} & \cdots & S_{n\lambda-400} \\ \vdots & \cdots & \vdots \\ S_{1\lambda-700} & \cdots & S_{n\lambda-700} \end{pmatrix}$$

That is, in the present example, data are obtained, for example, for 61 wavelength points between 400 to 700 nm. Thus, Formula 11 becomes matrix data of 61 × n. The principal component analysis in this case is to obtain characteristic values of the covariance matrix (n × n) shown in Formula 12 below and eigenvectors.

【Formula 12】

$$(\tilde{\$}\$)$$

where $\tilde{\$}$ is the transposed matrix of $ (same applies hereinafter)

Where one characteristic value is represented by $\lambda$ and the eigenvector therefor is represented by Formula 13 below, then Formula 14 below is satisfied.

【Formula 13】
$$x(n \times 1)$$

【Formula 14】
$$(\tilde{\$}\$)x = \Lambda x$$

In this example, the rank of

【Formula 15】
$$\$$$

is three, so that the number of $\lambda$ that satisfies Formula 14 above whose value is not 0 (zero) is three. These are assumed to be $\lambda 1$, $\lambda 2$, and $\lambda 3$ in which it is assumed that $\lambda 1 > \lambda 2 > \lambda 3$, and

【Formula 16】
$$x \text{ is assumed to be } x_1, x_2, \text{ and } x_3$$

Here, it is assumed that

【Formula 17】
$$X_1$$

corresponding to $\lambda 1$ is the first principal component. Thus, in this example, the reflection spectrum representing a region of interest to which a plurality of points is specified is obtained as

【Formula 18】
$$\$ x_1$$

That is,

【Formula 19】
$$\$ : 61 \times n \qquad x_1 : n \times 1$$

so that Formula 19 produces 61×1 data which indicate the reflection spectrum representing the region of interest. As described above, the use of principal component analysis allows the estimation of a highly reliable reflection spectrum.

In the present embodiment, signal processing circuit 32 has a not shown memory in which one set of data of Formula 19 is stored. When a set of reflection spectrum data newly estimated by reflection spectrum calculation means 100 (data of Formula 19) is inputted, signal processing circuit 32 combines the two sets of data and outputs an image signal representing both of them at the same time. Consequently, two reflection spectra SPr and SP of corresponding regions of interest are displayed in reflection spectrum display area 34p of monitor 34, as shown in Figure 3D.

In this case, for example, while displaying reflection spectrum SPr of a region of interest known to be normal, if reflection spectrum SP which is distinctly different from reflection spectrum SPr is displayed, the region of interest from which the latter reflection spectrum is obtained may be determined to be a lesion, so that advantageous effects in assisting in medical diagnosis are significant.

Further, the electronic endoscope apparatus of the present embodiment is configured so as to be capable of generating a spectroscopic image signal representing a spectroscopic image of a specified wavelength. This allows to find out a seemingly abnormal region of interest from a displayed reflection spectrum, and then to generate and display a detailed spectroscopic image of the region of interest. This may avoid the generation of many useless spectroscopic images and the efficiency of medical diagnosis may be improved.

Still further, in the present embodiment, monitor 34 as the image display means for displaying a spectroscopic image is also used as the means for displaying an estimated reflection spectrum, so that the cost of the electronic endoscope apparatus may be kept low. But it is not limited to the means which performs the both functions, and those dedicated to the respective types of displays may be used.

**Claims**

1. An electronic endoscope apparatus which includes a light source (14) for emitting white light on an observation object, a color imaging device (15) for imaging the observation object illuminated by the white light, and an image display means (34) for displaying a color image of the observation object from RGB three color image signals based on output of the color imaging device (15), **characterized in that** the apparatus comprises:

   a position specifying means (101) for specifying a desired region of interest in the color image displayed on the image display means (34);
   a calculation means (100) for estimating a reflection spectrum of the observation object with respect to the region of interest specified by the position specifying means (101) by performing arithmetic operations between RGB three color image signals of the region of interest and predetermined matrix data based on foresight information; and
   a spectrum display means (34) for displaying the reflection spectrum by receiving output of the calculation means (100), **characterized in that**: the position specifying means (101) is configured so as to be capable of specifying a plurality of points in a region of interest; and the calculation means (100) is configured to perform a principal component analysis on a plurality of estimated reflection spectra with respect to each of the plurality of points and to output information representing a reflection spectrum of the first principal component obtained by the analysis to the spectrum display means (34).

2. The electronic endoscope apparatus as claimed in claim 1,
   wherein the image display means (34) doubles as the spectrum display means (34).

3. The electronic endoscope apparatus as claimed in claim 1 or 2, **characterized in that**:

   the apparatus further comprises a storage means (32) for storing information representing the reflection spectrum of the observation object with respect to the region of interest obtained by the calculation means (100) ; and
   the spectrum display means (34) is configured so as to be capable of displaying the reflection spectrum represented by the information sent from the storage means (32) together with another reflection spectrum newly estimated by the calculation means (100) .

4. The electronic endoscope apparatus as claimed in any of claims 1 to 3, wherein the apparatus further comprises a spectroscopic image generation circuit (29) for generating spectroscopic image signals ($\lambda 1s$, $\lambda 2s$, and $\lambda 3s$) representing a spectroscopic image of a specified wavelength by performing arithmetic operations between the RGB three color image signals based on output of the color imaging device (15) and predetermined matrix data ($k_{1r}$, $k_{1g}$, $k_{1b}$, $k_{2r}$, $k_{2g}$, $k_{2b}$, $k_{3r}$, $k_{3g}$, $k_{3b}$).

**Patentansprüche**

1. Elektronische Endoskopvorrichtung, die eine Lichtquelle (14) zum Emittieren von weißem Licht auf ein Betrachtungsobjekt, eine Farbbildgebungseinrichtung (15) zum Abbilden des mit dem weißen Licht beleuchteten Betrachtungsobjekts, und eine Bildanzeigeeinrichtung (34) zum Anzeigen eines Farbbilds des Betrachtungsobjekts anhand von drei RGB-Farbbildsignalen basierend auf einem Ausgangssignal der Farb-Bildgebungseinrichtung (15) enthält, **dadurch gekennzeichnet, dass** die Vorrichtung aufweist:

   eine Positionsspezifiziereinrichtung (101) zum Spezifizieren einer gewünschten interessierenden Zone in dem auf der Bildanzeigeeinrichtung (34) angezeigten Farbbild;
   eine Berechnungseinrichtung (100) zum Abschätzen eines Reflexionsspektrums des Betrachtungsobjekts bezüglich der von der Positionsspezifiziereinrichtung (101) spezifizierten interessierenden Zone durch Ausführen arithmetischer Operationen zwischen den drei RGB-Farbbildsignalen der interessierenden Zone und vorbestimmten Matrixdaten, basierend auf Vorausschau-Information; und
   eine Spektrum-Anzeigeeinrichtung (34) zum Anzeigen des Reflexionsspektrums durch Empfangen von Ausgangssignalen der Berechnungseinrichtung (100),
   **dadurch gekennzeichnet, dass**
   die Positionsspezifiziereinrichtung (101) konfiguriert ist, um in der Lage zu sein, eine Mehrzahl von Punkten in einer interessierenden Zone zu spezifizieren; und
   die Berechnungseinrichtung (100) konfiguriert ist zum Ausführen einer Hauptkomponentenanalyse bezüglich einer Mehrzahl abgeschätzter Reflexionsspektren in Bezug auf jeden der mehreren Punkte, und zum Ausgeben von Information, die ein Reflexionsspektrum der ersten, durch die Analyse erhaltenen Hauptkomponente repräsentiert, an die Spektrum-Anzeigeeinrichtung (34).

2. Elektronische Endoskopvorrichtung nach Anspruch 1, bei der die Bildanzeigeeinrichtung (34) gleichzeitig als Spektrum-Anzeigeeinrichtung (34) fungiert.

3. Elektronische Endoskopvorrichtung nach Anspruch 1 oder 2, **dadurch gekennzeichnet, dass**
   die Vorrichtung außerdem eine Speichereinrichtung (32) aufweist, um Information zu speichern, welche das Reflexionsspektrum des Betrachtungsobjekts bezüglich der interessierenden Zone, gewonnen durch die Berechnungseinrichtung (100), repräsentiert; und
   die Spektrum-Anzeigeeinrichtung (34) konfiguriert ist, um in der Lage zu sein, das durch die von der Speichereinrichtung (32) gesendete Information repräsentierte Reflexionsspektrum zusammen mit einem weiteren, neuerlich von der Berechnungseinrichtung (100) abgeschätzten Reflexionsspektrum anzuzeigen.

4. Elektronische Endoskopvorrichtung nach einem der Ansprüche 1 bis 3, wobei die Vorrichtung außerdem aufweist: eine Spektroskopbild-Erzeugungsschaltung (29) zum Erzeugen von spektroskopischen Bildsignalen ($\lambda1s$, $\lambda2s$ und $\lambda3s$), die ein spektroskopisches Bild einer spezifizierten Wellenlänge repräsentiert, indem arithmetische Operationen zwischen den drei RGB-Farbbildsignalen basierend auf Ausgangssignalen der Farb-Bildgebungseinrichtung (15) und vorbestimmten Matrixdaten ($k_{1r}$, $k_{1g}$, $k_{1b}$, $k_{2r}$, $k_{2g}$, $k_{2b}$, $k_{3r}$, $k_{3g}$, $k_{3b}$) ausgeführt werden.


**Revendications**

1. Appareil endoscope électronique incluant une source de lumière (14) pour émettre de la lumière blanche sur un objet d'observation, un dispositif d'imagerie couleur (15) pour former l'image de l'objet d'observation éclairé par la lumière blanche et un moyen d'affichage d'image (34) pour afficher une image en couleur de l'objet d'observation à partir de trois signaux d'image couleur RGB basés sur la sortie du dispositif d'imagerie couleur (15), **caractérisé en ce que** l'appareil comprend :

   un moyen de spécification de position (101) pour spécifier une région d'intérêt désirée dans l'image en couleur affichée sur le moyen d'affichage d'image (34) ;
   un moyen de calcul (100) pour estimer le spectre de réflexion de l'objet d'observation par rapport à la région d'intérêt spécifiée par le moyen de spécification de position (101) en effectuant des opérations arithmétiques entre les trois signaux d'image couleur RGB de la région d'intérêt et des données de matrice prédéterminées basées sur des informations de visée avant ; et
   un moyen d'affichage de spectre (34) pour afficher le spectre de réflexion par réception de la sortie du moyen de calcul (100),

## EP 2 105 086 B1

**caractérisé en ce que** :

le moyen de spécification de position (101) est configuré de façon à être capable de spécifier une pluralité de points dans une région d'intérêt, et
le moyen de calcul (100) est configuré pour effectuer une analyse de composante principale sur une pluralité de spectres de réflexion estimés par rapport à chaque pluralité de points et pour délivrer en sortie au moyen d'affichage de spectre (34) des informations représentant le spectre de réflexion de la première composante principale, obtenu par l'analyse.

2. Appareil endoscope électronique selon la revendication 1, dans lequel le moyen d'affichage d'image (34) est doublé en tant que moyen d'affichage d'image (34).

3. Appareil endoscope électronique selon la revendication 1 ou 2, **caractérisé en ce que** :

l'appareil comprend en outre un moyen d'enregistrement (32) pour enregistrer des informations représentant le spectre de réflexion de l'objet d'observation par rapport à la région d'intérêt, obtenu par le moyen de calcul (100) ; et
le moyen d'affichage de spectre (34) est configuré de façon à être capable d'afficher le spectre de réflexion représenté par les informations envoyées par le moyen d'enregistrement (32) en même temps qu'un autre spectre de réflexion nouvellement estimé par le moyen de calcul (100).

4. Appareil endoscope électronique selon l'une quelconque des revendications 1 à 3, dans lequel l'appareil comprend en outre un circuit de génération d'images spectroscopiques (29) pour générer des signaux d'images spectroscopiques ($\lambda 1s$, $\lambda 2s$ et $\lambda 3s$) représentant une image spectroscopique d'une longueur d'onde spécifiée en effectuant des opérations arithmétiques entre les trois signaux d'image couleur RGB basés sur la sortie du dispositif d'imagerie couleur (15) et des données de matrice prédéterminées ($k_{1r}$, $k_{1g}$, $k_{1b}$, $k_{2r}$, $k_{2g}$, $k_{2b}$, $k_{3r}$, $k_{3g}$, $k_{3b}$).

# FIG.1

Blocks and labels:

- POSITION SPECIFYING MEANS (101)
- MONITOR (34)
- IMAGE RECORDING UNIT (45)
- REFLECTION SPECTRUM CALCULATION MEANS (100)
- D/A (33)
- SIGNAL PROCESSING CIRCUIT (32)
- MATRIX DATA (36)
- OPERATION PANEL (41)
- IMAGE RECORDING CONTROLLER (42)
- RsGsBs → Y/C (31)
- MODE SELECTOR (30)
- MICRO COMPUTER (35)
- COLOR SPACE CONVERSION CIRCUIT (29)
- λ1s λ2s λ3s
- R G B
- V/C → RGB (28)
- INPUT UNIT (43)
- DSP (25)
- A/D (18)
- MICRO COMPUTER (20)
- CDS/AGC (17)
- CCD DRIVE CIRCUIT (16)
- (15) (23) (24)
- (10) (12)
- 14, 14a, 14b, 14c, 14d

$$
\begin{bmatrix} \lambda_{1s} \\ \lambda_{2s} \\ \lambda_{3s} \end{bmatrix}
=
\begin{bmatrix} k_{1r} & k_{1g} & k_{1b} \\ k_{2r} & k_{2g} & k_{2b} \\ k_{3r} & k_{3g} & k_{3b} \end{bmatrix}
\times
\begin{bmatrix} R \\ G \\ B \end{bmatrix}
$$

# FIG.2

41

41c  1 5 10
      30 25 20 15

SPECTROSCOPIC IMAGE
GENERATION SWITCH
                    41j

| SET | λ1 | λ2 | λ3 |
|:---:|:---:|:---:|:---:|
| △▽ | △▽ | △▽ | △▽ |

MODE CHANGE
SWITCH

41a          41b          41d

| | λ1 | λ2 | λ3 |
|---|:---:|:---:|:---:|
| a. STANDARD (BASIC) | 400 (λ1) | 500 (λ2) | 600 (λ3) |
| b. VESSEL B1 | 470 | 500 | 670 |
| c. VESSEL B2 | 475 | 510 | 685 |
| d. TISSUE E1 | 440 | 480 | 520 |
| e. TISSUE E2 | 480 | 510 | 580 |
| f. HEMOGLOBIN | 400 | 430 | 475 |
| g. BLOOD-CAROTENE | 415 | 450 | 500 |
| h. BLOOD-CYTOPLASM | 420 | 550 | 600 |
| | (λ1) | (λ2) | (λ3) |

# FIG.3A

34

34p

34s

# FIG.3B

b. VESSEL B1

λ1    λ2    λ3

470    500    670

34s

# FIG.3C

d. TISSUE E1

λ1    λ2    λ3

400    500    600    700

34s

# FIG.3D

REFLECTION LIGHT INTENSITY

SP

SPr

34p

WAVELENGTH

FIG.4

FIG.5

# FIG.6

| λ1 | λ2 | λ3 |
|----|----|----|
| 400 | 500 | 600 |
| ↓ | ↓ | ↓ |
| 405 | 510 | 620 |
| ↓ | ↓ | ↓ |
| 410 | 520 | 640 |
| ↓ | ↓ | ↓ |
| 415 | 530 | 660 |
| ↓ | ↓ | ↓ |

# FIG.7

(MONOCHROME MODE, SINGLE WAVELENGTH SET)

| λ1 | λ2 | λ3 |
|----|----|----|
| 470 | 470 | 470 |
| : | | |
| 500 | 500 | 500 |
| : | | |
| 530 | 530 | 530 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7096005 A
- JP 2003093336 A
- JP 2004202217 A
- EP 1994875 A1

### Non-patent literature cited in the description

- **Yoichi MIYAKE.** Introduction to Multi-spectral Imaging. University of Tokyo Press, 2006, 21-35171-173
- **N. TSUMURA et al.** Estimation of Spectral Reflectances from Multi-Band Images by Multiple Regression Analysis. *Optics,* 1998, vol. 27 (7), 384-391